# EUROPÄISCHE PATENTSCHRIFT

(11) **EP 1 713 421 B1**
(45) Veröffentlichungstag und Bekanntmachung des Hinweises auf die Patenterteilung: **01.12.2010**
(21) Anmeldenummer: 04710828.7
(22) Anmeldetag: 13.02.2004
(51) Int. Cl.: A61F 2/44

(54) **ZWISCHENWIRBELIMPLANTAT ZUR SPONDYLODESE DER LENDENWIRBELSÄULE**
INTERVERTEBRAL IMPLANT FOR SPONDYLODESIS OF A LUMBAR VERTEBRAL COLUMN
IMPLANT INTERVERTEBRAL DE SPONDYLODESE DE LA COLONNE VERTEBRALE LOMBAIRE

(43) Veröffentlichungstag der Anmeldung: 25.10.2006
(73) Patentinhaber: Copf jun., Franz, 70184 Stuttgart (DE)
(72) Erfinder: Copf jun., Franz, 70184 Stuttgart (DE)
(74) Vertreter: Schwanhäusser, Gernot
(86) Internationale Anmeldenummer: PCT/EP2004/001359
(87) Internationale Veröffentlichungsnummer: WO 2005/082292

(56) Entgegenhaltungen:
- EP-A- 1 103 235
- JP-A- 2001 238 891
- US-A1- 2003 045 938

## Beschreibung

Die Erfindung betrifft ein Zwischenwirbelimplantat gemäß des Oberbegriffes von Anspruch 1.

Ein zwischenwirbelimplantat der o. g. Art ist in der EP 1 103 235 beschrieben. Dieses verzichtet bewusst auf die früher bekannte hülsenartige Form von Zwischenwirbelimplantaten und verwendet statt dessen einen Zentralabschnitt mit schmalen Streben, die möglichst große Fenster offen lassen. Für die in das Zwischenwirbelimplantat eingebrachte Spongiosa kann auf diese Weise ein großflächiger direkter Weg von einem wirbelkörper zum anderen freigegeben werden, was einen besonders guten Kraftschluß ermöglicht und die Knochenbildung beschleunigt. Außerdem ist bei derartigen Zwischenwirbelimplantaten eine Röntgegnkontrolle während und nach dem Eingriff besser möglich.

Bei dem bekannten Zwischenwirbelimplantat besitzt der Kopf einen zylindrischen Abschnitt, der das Außengewinde trägt, sowie einen sich im Endbereich anschließenden konischen, gewindefreien Abschnitt. Mit diesem Zwischenwirbelimplantat ist es nicht risikolos möglich, eine vollständige Distraktion insbesondere der ventralen Zirkumferenz der Wirbelkörper vorzunehmen, da die Gefahr einer Verletzung der benachbarten Hohlvene und der Aorta nicht völlig auszuschließen ist.

Aus der WO 95/25487 ist ein Zwischenwirbelimplantat bekannt, welches annähernd die Grundform eines länglichen Rotationsellipsoids hat. Ein an den Bereich des größten Umfangs angrenzender Teil der vorderen Hälfte des Zwischenwirbelimplantats ist mit einem Außengewinde versehen. Ansonsten ist das Zwischenwirbelimplantat außen glatt und frei von scharfen Kanten. Das Zwischenwirbelimplantat kann über einen posterioren lateralen Zugang in den Zwischenwirbelraum eingesetzt werden. Der mit dem Gewinde versehene Außenabschnitt liegt dabei an den leicht konkav gewölbten Innenflächen der angrenzenden Wirbel an.

Aufgabe der vorliegenden Erfindung ist es, ein Zwischenwirbelimplantat der eingangs genannten Art so auszugestalten, daß es gleichzeitig zur Distraktion, insbesondere auch im ventralen Bereich der Wirbelkörper, geeignet ist.

Diese Aufgabe wird erfindungsgemäß durch die Merkmale des kennzeichnenden Teiles von Anspruch 1 gelöst.

Das erfindungsgemäße Zwischenwirbelimplantat kann mit dem Außengewinde seines Kopfes in die Compacta an der ventralen Zirkumferenz der Wirbelkörper eingedreht werden, wobei aufgrund der Konizität eine entsprechende Distraktion der Wirbelkörper stattfindet. Die Schutzerhebung des Kopfes wird dabei über die ventrale Kante der Wirbelkörper hinaus bewegt, kann aber wegen ihrer Freiheit von scharfen Kanten die benachbarten großen Gefäße nicht verletzen sondern drängt diese allenfalls stumpf etwas von den Wirbelkörpern weg. Das erfindungsgemäße Zwischenwirbelimplantat läßt sich exakt auf die Wirbelkörperränder positionieren.

Der konische Abschnitt des Kopfes sollte eine axiale Erstreckung von mindestens 7 mm besitzen, um ausreichende Kräfte auf die Wirbelkörper an deren ventraler Zirkumferenz während des Distraktionsvorganges übertragen zu können.

Aus dem gleichen Grunde empfiehlt es sich, daß das Außengewinde auf dem Kopf mindestens drei volle Gewindegänge aufweist.

Die Schutzerhebung des Kopfes sollte eine axiale Erstreckung von mindestens 5 mm aufweisen, um sicherzustellen, daß tatsächlich alle scharfen Kanten, wozu auch Gewindegänge gehören können, stets eine ausreichende Entfernung von den benachbarten Gefäßen besitzen.

Die Schutzerhebung kann die Form einer geschlossenen Kappe besitzen oder auch ringförmig sein und dabei eine mittlere Durchgangsöffnung aufweisen.

Die Grundstruktur des erfindungsgemäßen Zwischenwirbelimplantats besitzt vorzugsweise eine axiale Erstreckung von mindestens 5 mm. Dabei wird der anatomischen Tatsache Rechnung getragen, daß die Kompakta an der dorsalen Seite der Wirbelkörper dünner als an der ventralen Seite ist.

Die Grundstruktur kann insbesondere auch eine Platte sein.

Sie sollte eine Angriffseinrichtung für ein Eindrehinstrument aufweisen, wobei eine solche Ausführungsform bevorzugt wird, die sich nur unter aktiver Wirkung des Operateurs an das Eindrehinstrument ankoppeln und ebenfalls nur unter aktiver Mitwirkung des Operateurs wieder lösen läßt. Derartige Angriffseinrichtungen sind bekannt.

Weitgehend unabhängig von den individuellen Patientendaten sollte der von der konischen Mantelfläche des konischen Abschnittes des Kopfes und von einer gedachten koaxialen Kreiszylinder-Mantelfläche eingeschlossene Winkel (α) zwischen 10° und 20°, bevorzugt bei etwa 15°, liegen.

Eine besonders vorteilhafte Ausführungsform des erfindungsgemäßen Zwischenwirbelimplantates zeichnet sich dadurch aus, daß der Zentralabschnitt und die Grundstruktur gegensinnig zum konischen Abschnitt des Kopfes konisch sind. Die Konizität des Zentralabschnittes und der Grundstruktur bestimmen somit die Lordose der beiden Wirbelkörper, zwischen die das Zwischenwirbelimplantat eingesetzt wird.

Entsprechend den allgemeinen anatomischen Gegebenheiten genügt es im allgemeinen, wenn für den Zentralabschnitt und die Grundstruktur zwei Konizitätswinkel wahlweise bereitgehalten werden: Der von der konischen Mantelfläche des Zentralabschnittes und der Grundstruktur und von einer gedachten koaxialen Kreiszylinder-Mantelfläche eingeschlossene Winkel (β) sollte bei einer Ausführungsform etwa 3° und bei einer anderen Ausführungsform etwa 6° betragen.

Das Zwischenwirbelimplantat kann aus unterschiedlichsten Materialien, insbesondere aus rostfreiem Stahl, Kohlenstoff-Keramik-Material, Aluminiumlegierungen, Titan, Kunststoff, dabei insbesondere Polyisocyanat, oder dgl. bestehen.

Das erfindungsgemäße Zwischenwirbelimplantat kann jedoch auch aus bioresorbierbarem Material, insbesondere Polyaktid, hergestellt werden.

Wenn das Zwischenwirbelimplantat in mindestens einem axialen Bereich eine nicht rotationssymmetrische Querschnittsform aufweist, ist es nach dem Eindrehen gegen eine unbeabsichtigte Verdrehung gesichert. Besonders günstig ist es dabei, wenn die Grundstruktur selbst, die mit dem dorsalen Bereich der Compacta der Wirbelkörper zusammenwirkt, eine nicht rotationssymmetrische Querschnittsform aufweist.

Besonders gefäßschonend ist das Zwischenwirbelimplantat, wenn seine Schutzerhebung poliert ist.

Das erfindungsgemäße Implantat kann z. B. auf folgende Weise zur Spondylodese.der Lendenwirbelsäule eingesetzt werden:
a) der Zugang zum Wirbelzwischenraum wird mikrochirurgisch von der dorsalen Seite aus unter Teilentfernung der kleinen Wirbelgelenke eröffnet;
b) die Knorpelanteile der Deck- und Bodenplatte des Wirbelzwischenraumes werden entfernt;
c) in den dorsalen Bereich des Wirbelzwischenraumes wird ein Gewinde eingeschnitten;
d) der Wirbelzwischenraum wird mit Hilfe eines Distraktionsinstrumentariums vordistrahiert;
e) die Vordistraktion wird mit Hilfe eines Distanzelementes aufrecht erhalten und das Distraktionsinstrumentarium entfernt;
f) in das Gewinde des Wirbelzwischenraumes wird ein erstes Zwischenwirbelimplantat eingedreht;
g) die Schritte a) bis f) werden für ein zweites Zwischenwirbelimplantat wiederholt, das im Abstand vom ersten Zwischenwirbelimplantat eingesetzt wird.

Dieses chirurgische Verfahren schont den Patienten aufgrund seiner mikrochirurgischen Ausgestaltung und seines Angriffes von der dorsalen Seite her und setzt nur sehr kleine Wunden. Die Infektionsgefahr ist daher erheblich reduziert. Der Patient ist sehr viel geringeren Schmerzen ausgesetzt und die Verweilzeit im Krankenhaus ist erheblich kürzer.

Sofern der Patient nicht bereits voroperiert ist, kann es erforderlich werden, nach dem Schritt a) das Ligamentum Flavum von außen nach innen einzudünnen. Ein innerer Bereich bleibt dabei jedoch erhalten, so daß eine Narbenbildung weitgehend vermieden wird.

Nach dem Schritt b) können die freigelegten Knochenanteile angefrischt, also oberflächlich abgeschabt werden, was das Verwachsen mit in den Wirbelzwischenraum eingebrachter Spongiosa erleichtert.

Die Zwischenwirbelimplantate selbst sollten vor dem Einführen in den Wirbelzwischenraum mit Spongiosa gefüllt sein.

Auch der Wirbelzwischenraum selbst wird vorzugsweise spätestens vor dem Einführen des zweiten Zwischenwirbelimplantates mit Spongiosa gefüllt.

Besonders hilfreich ist dieses Verfahren, wenn das Zwischenwirbelimplantat unter Distraktion insbesondere des ventralen Bereiches so weit eingedreht wird, bis die Übergangslinie zwischen der Schutzerhebung und dem konischen Bereich des Kopfes auf der Höhe der Scheitelpunkte in der seitlichen Aufnahme der ventralen Zirkumferenz der Wirbelkörper liegt. Bei dieser Ausgestaltung des Verfahrens läßt sich also risikolos insbesondere auch der ventrale Bereich der Wirbelkörper in der erforderlichen Weise distrahieren.

Beim Eindrehen des Zwischenwirbelimplantates kann das Drehmoment gemessen werden. Wird ein vorgegebener Wert des Drehmomentes unterschritten, bedeutet dies, daß der maximale Durchmesser des verwendeten Zwischenwirbelimplantates zu klein und die erreichte Distraktion nicht ausreichend ist. Es wird dann ein anderes Zwischenwirbelimplantat mit größerem maximalen Durchmesser gewählt.

Ein Ausführungsbeispiel der Erfindung wird nachfolgend anhand der Zeichnung näher erläutert; es zeigen
- Figur 1: ein Zwischenwirbelimplantat in Seitenansicht mit Bemaßung;
- Figur 2: die Unteransicht des Zwischenwirbelimplan- tates von Figur 1;
- Figur 3: schematisch in Draufsicht zwei nebeneinander zwischen zwei Wirbelkörpern implantierte Zwi- schenwirbelimplantate;
- Figur 4: in etwas größerem Maßstab ein Zwischenwirbel- implantat in Seitenansicht zwischen zwei im Schnitt dargestellten Wirbelkörpern.

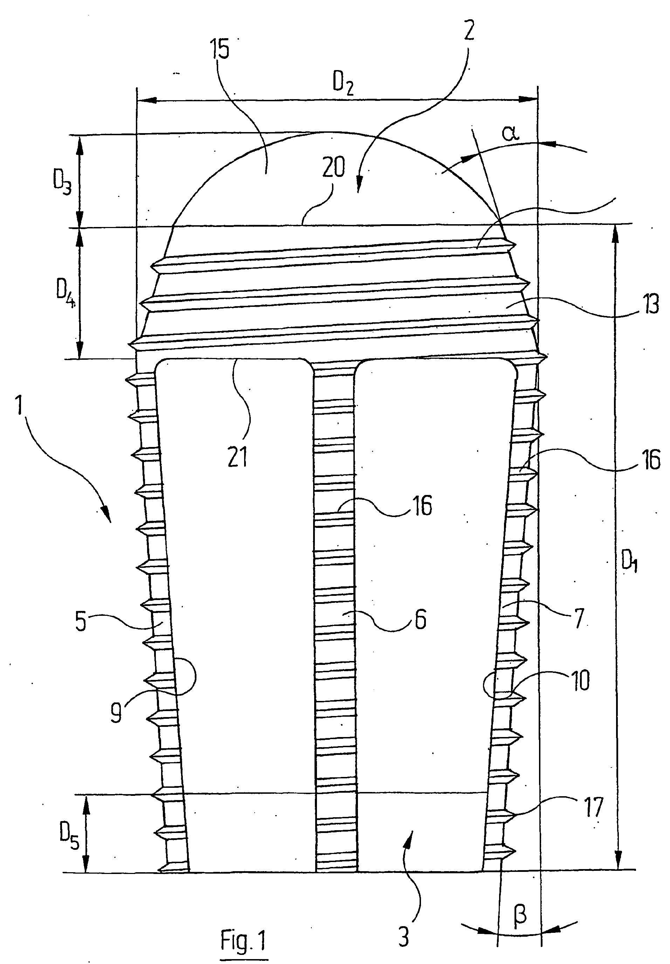

Zunächst wird auf Figur 1 Bezug genommen. Diese zeigt ein Zwischenwirbelimplantat, das insgesamt mit dem Bezugszeichen 1 gekennzeichnet ist. Es umfasst einen in implantiertem Zustand ventral zeigenden Kopf 2 sowie eine in implantiertem Zustand dorsal zeigende Grundplatte 3, die durch vier schmale Stege 5, 6, 7 und 8 miteinander verbunden sind. Zwischen den Stegen 5, 6, 7, 8, dem Kopf 2 und der Grundplatte 3 verbleiben großflächige Fenster 9, 10, 11, 12.

Der Kopf 2 des Zwischenwirbelimplantates 1 umfasst einen konischen Abschnitt 13, der ein Außengewinde 14 trägt, sowie eine sich an den konischen Abschnitt 13 anschließenden, das ventrale Ende des Zwischenwirbelimplantates 1 bildende, von außen her gesehen konvexe Schutzkappe 15. Die Schutzkappe 15, die im dargestellten Ausführungsbeispiel eine Kugelkalottenfläche ist, ist aus Gründen, die weiter unten deutlich werden, poliert; sie weist also insbesondere keinerlei scharfe Kanten auf. Die Pfeilhöhe D₃ der Schutzkappe 15 beträgt mindestens 4 bis 5 mm; der Grund hierfür wird ebenfalls weiter unten deutlich. Der konische Abschnitt 13 besitzt eine axiale Länge D_{4'} die 7 mm nicht unterschreiten sollte. Das Außengewinde 14 besitzt innerhalb des konischen Abschnittes 3 mindestens drei vollständige Gänge. Der Winkel α, den die Konusmantelfläche des konischen Abschnittes 13 mit einer gedachten Kreiszylinder-Mantelfläche einschließt und dem halben Konuswinkel entspricht, liegt zwischen 10 und 20°, bevorzugt bei etwa 15°.

Auch die Stege 5, 6, 7, 8 liegen auf einer gedachten Kreiskonusfläche, die sich jedoch in entgegengesetztem Sinne wie der konische Bereich 13 des Kopfes 2 zum in Figur 1 unten liegenden dorsalen Ende des Zwischenwirbelimplantates 1 verjüngt. Der Winkel β, den die Konusmantelfläche dieses gedachten Konuses mit einer Kreiszylinder-Mantelfläche einschließt und dem halben Konuswinkel entspricht, liegt entweder bei etwa 6° oder bei etwa 3°, je nach dem, zwischen welchen Wirbelkörpern das Zwischenwirbelimplantat 1 eingesetzt werden soll. Auch die Stege 5, 6, 7, 8 tragen ein Außengewinde 15, welches als Fortsetzung des Außengewindes 14 auf dem konischen Bereich 13 des Kopfes 2 ausgebildet ist.

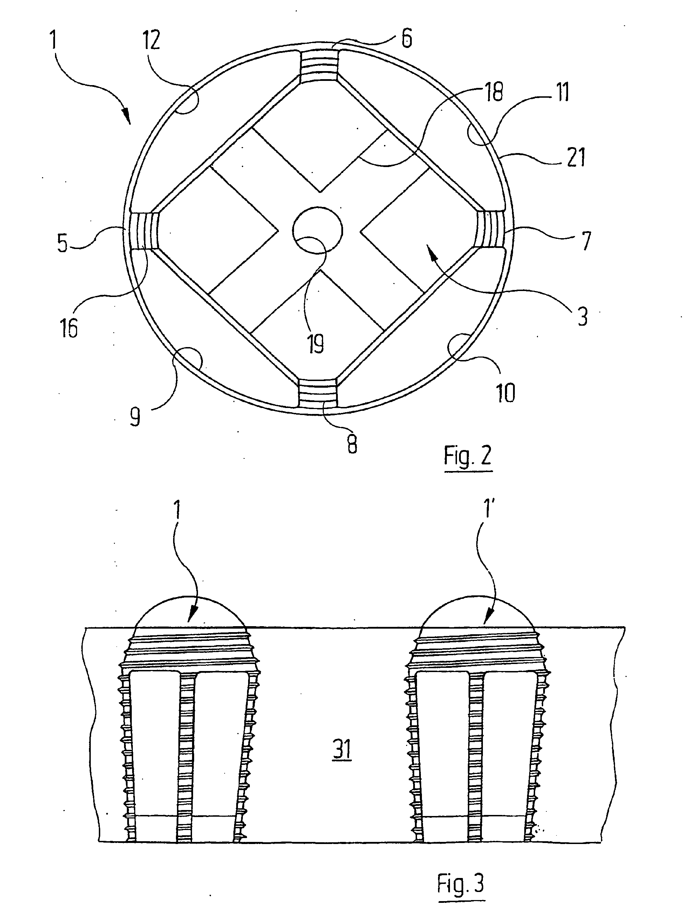

Die Stege 5, 6, 7, 8 gehen einstückig in die abgerundeten Ecken der Grundplatte 3 über, wie dies insbesondere die Figur 2 zeigt. Die Unteransicht der Grundplatte 3 ist im übrigen etwa quadratisch. Das Außengewinde 16 der Stege 5, 6, 7, 8 setzt sich in einem Außengewinde 17 auf den abgerundeten Ecken der Grundplatte 3 fort.

In die Unterseite der Grundplatte 3 ist, wie der Figur 2 zu entnehmen ist, eine Angriffseinrichtung für ein Eindrehinstrument eingearbeitet, die im dargestellen Falle die Form einer Kreuznut 18 besitzt. Das zugehörige, in der Zeichnung jedoch nicht dargestellte Eindrehinstrument besitzt einen komplementären kreuzförmigen, sich axial erstreckenden Vorsprung und kann in eine Rasteinrichtung 19 eingeklickt werden, die in der Zeichnung schematisch als Kreisbohrung dargestellt ist. Die Rasteinrichtung 19 ist so ausgestaltet, daß das Herstellen einer Verbindung zwischen dem Eindrehinstrument und der Kreuznut 18 ein aktives Handeln des Operateurs erfordert und das umgekehrt ein Lösen des Eindrehinstruments von der Grundplatte 3 nur unter aktivem Handeln des Operateurs und nicht unabsichtlich erfolgen kann. Verbindungen dieser Art sind an und für sich bekannt und brauchen daher nicht näher beschrieben zu werden.

Die axiale Erstreckung D₅ der Grundplatte 3 sollte 4 mm nicht unterschreiten.

Soweit bisher beschrieben, unterscheidet sich das Zwischenwirbelimplantat 1 im wesentlichen nicht von Patient zu Patient. Individuelle Patientendaten fließen in die Abmessungen des Zwischenwirbelimplantates 1 jedoch in folgender Weise ein und müssen präoperativ den CT- bzw. Röntgenaufnahmen entnommen werden:

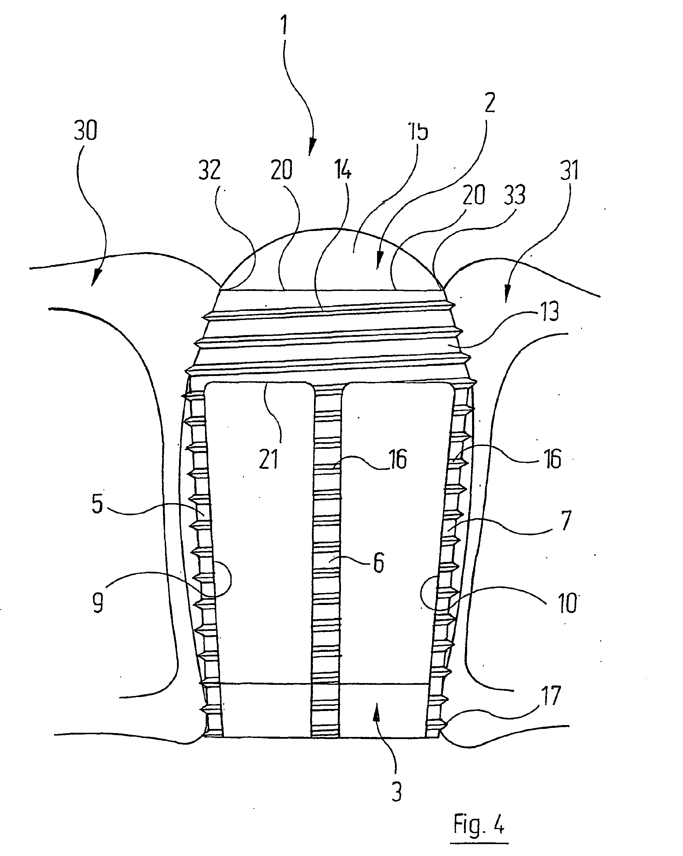

Die axiale Entfernung D₁ zwischen der dorsalen, äußeren Stirnseite der Grundplatte 3 und der Übergangslinie 20 zwischen dem konischen Bereich 13 des Kopfes 2 und der polierten Schutzkappe 15 entspricht, wie dies insbesondere der Figur 4 zu entnehmen ist, dem Abstand zwischen den dorsalen Außenkanten der zu verbindenden Wirbelkörper 30, 31 und den gegenüberliegenden Scheitelpunkten 32, 33 der ventralen Kanten der Wirbelkörper 30, 31 in der seitlichen Aufnahme. Die Implantationsposition des Zwischenwirbelimplantates 1 ist also so, daß die dorsale äußere Stirnfläche etwa mit den dorsalen Kanten der Wirbelkörper 30, 31 fluchtet, während die Übergangslinie 20 am Kopf 2 des Zwischenwirbelimplantates 1 die Scheitelpunkte 32, 33 der beiden Wirbelkörper 30, 31 verbindet. Die Schutzkappe 15 des Kopfes 2 ragt dabei ventral über die ventralen Kanten der Wirbelkörper 30, 31 hinaus.

Das Zwischenwirbelimplantat 1 kann aus unterschiedlichen Materialien bestehen. Zum einen kommen, wie dies bereits in der oben erwähnten DE 199 57 339 C2 beschrieben ist, nicht rostender Stahl oder Kohlenstoff-Keramik-Material in Frage. Als permanent im Körper verbleibende Materialien sind auch Aluminiumlegierungen, Titan sowie gewisse Kunststoffe, insbesondere Polyisocyanat (PIC) geeignet. Verwendbar sind aber auch bioresorbierbare Materialien, wie insbesondere Polyaktid, also Kopolymere aus Poly (L-Laktid-co-D, L-Laktid), wie diese beispielsweise unter dem Handelsnamen Telamon vertrieben werden.

Für unterschiedliche Patienten müssen also Sätze von Zwischenwirbelimplantaten 1 bereitgehalten werden, die sich in der Größe der Abmessung D1 unterscheiden. Im allgemeinen reicht es aus, hier Abstufungen von 2 mm vorzusehen, wobei für die Praxis insbesondere Abmessungen D1 von 28, 30, 32, 34, 36 mm in Frage kommen.

Der größte Durchmesser D2 des Zwischenwirbelimplantates 1 tritt, wie sich aus der obigen Beschreibung ergibt, an der Übergangslinie 21 zwischen dem Kopf 2 und den Streben 5, 6, 7 auf. Er entspricht der gewünschten Distraktion zwischen den benachbarten Wirbelkörpern 30, 31 und kann dem Röntgenbild beispielsweise aus benachbarten, gesunden Segmenten entnommen werden. Auch bezuüglich der Abmessung D₂ ist somit ein ganzer Satz von Zwischenwirbelimplantaten 1 erforderlich, die vom Operateur bereitgehalten werden müssen. Erneut genügt eine Abstufung von 2 mm; in der Praxis kommen vor allem Abmessungen D₂ von 10, 12, 14 und 16 mm in Frage.

Bei einem in der Zeichnung nicht dargestellten Ausführungsbeispiel des Zwischenwirbelimplantates 1 ist das ventrale Ende nicht durch eine geschlossene Schutzkappe sondern durch einen von außen gesehen konvex geformten Ring mit einer Durchgangsöffnung gebildet, die nicht größer als 5 mm sein sollte. Die in ventraler Richtung zeigende Außenfläche dieses Schutzringes sollte ebenfalls poliert sein.

Der Operateur führt mit dem beschriebenen Zwischenwirbelimplantat 1, von dem jeweils zwei benötigt werden, in folgender Weise die Spondylodese durch:
Zunächst werden präoperativ aus den CT- bzw. Röntenaufnahmen die individuellen Daten D₁ und D₂ der zu verwendenden Zwischenwirbelimplantate 1 .ermittelt. Der Winkel β wird je nach der gewünschten Lordose der miteinander zu verbindenden Wirbelkörper gewählt.

Die Operation wird mikrochirurgisch von der dorsalen Seite her durchgeführt. Der erforderliche Zugang zum Wirbelzwischenraum wird unter Teilentfernung der kleinen Wirbelgelenke gewonnen, wobei bei nicht voroperierten Patienten das Ligamentum Flavum weitgehend geschont werden kann. Dieses braucht nur von außen nach innen etwas ausgedünnt werden, so daß eine innere Schicht erhalten bleibt. Auf diese Weise wird eine Narbenbildung vermieden.

Der Wirbelzwischenraum wird sodann sorgfältig von Knorpelanteilen der Deck- und Bodenplatte befreit. Die Knochenanteile werden dabei "angefrischt", d. h., oberflächlich dünn abgetragen, wobei ihre Tragfähigkeit nicht verletzt wird. Sodann wird mit Hilfe eines Distraktionsinstrumentariums, welches im wesentlichen an den dorsalen Wirbelkanten ansetzt, eine Vordistraktion der Wirbelkörper 30, 31 durchgeführt. Die so erreichte Vordistraktion wird durch eine mit zwei angesetzten, in den Wirbelzwischenraum eingeführten Distanzlaschen versehenen Hülse aufrecht erhalten, wenn nunmehr das Distraktionsinstrumentarium wieder entfernt wird.

Durch diese Hülse hindurch wird sodann im dorsalen Teil des Wirbelzwischenraumes ein Gewinde eingeschnitten. Durch die Hülse hindurch wird jetzt ein erstes Zwischenwirbelimplantat 1, mit dem Kopf 2 voraus, eingeführt und durch Verdrehen mit dem zuvor eingeschnittenen Gewinde der Wirbelkörper 30, 31 verschraubt, so daß es sich axial in ventraler Richtung bewegt. Das Zwischenwirbelimplantat 1 wurde zuvor mit Spongiosa gefüllt.

Das Zwischenwirbelimplantat 1 wird nunmehr so lange verdreht, bis das Außengewinde 14 am Kopf 2 des Zwischenwirbelimplantates 1 an der Compacta der ventralen Zirkumferenz der beiden Wirbelkörper 30, 31 zu liegen kommt. Beim weiteren Verdrehen des Zwischenwirbelimplantates 1 schneidet sich das Außengewinde 14 in die Compacta der ventralen Kanten der Wirbelkörper 30, 31 ein, wobei die konische Form des konischen Bereiches 13 des Kopfes 2 für eine entsprechende Distraktion der ventralen Kanten der Wirbelkörper 30, 31 sorgt. Die Eindrehbewegung des Zwischenwirbelimplantates 1 wird unter Röntgenbeobachtung so weit fortgesetzt, bis die Übergangslinie 20 in dem Bereich der Scheitelpunkte 32, 33 der beiden Wirbelkörper 30, 31 zu liegen kommt. Eine Weiterbewegung darf nicht erfolgen. Bis zu diesem Zeitpunkt hat die Schutzkappe 15 des Kopfes 2 möglicherweise die in diesem Bereich verlaufende Hohlvene und die Aorta etwas in ventraler Richtung weggedrückt, ohne diese jedoch verletzten zu können, da scharfe Kanten fehlen.

Nunmehr wird der verbleibende Wirbelzwischenraum mit Spongiosa angefüllt und in der oben schon beschriebenen Weise neben dem ersten Zwischenwirbelimplantat 1 ein zweites Zwischenwirbelimplantat 1' eingesetzt (vgl. Figur 3). Der Abstand zwischen den beiden Zwischenwirbelimplantaten 1, 1' sollte nicht kleiner als 1 cm sein. Auch das zweite Zwischenwirbelimplantat 1' wurde bereits vor dem Einsetzten mit Spongiosa gefüllt.

Beim Eindrehen der Außengewinde 14 der beiden Zwischenwirbelimplantate 1, 1' wird das Drehmoment gemessen, das hierfür erforderlich ist. Liegt dieses Drehmoment unter einem bestimmten Wert, ist dies ein Zeichen dafür, daß die Abmessung D₂ des aus dem Satz gewählten Zwischenwirbelimplantates 1 zu klein ist; das Zwischenwirbelimplantat 1 muß dann gegen ein solches ausgetauscht werden, dessen Abmessung D2 etwas größer ist.

Bei ausreichender Distraktion ohne ventrale Verschiebung der Wirbelkörper sind keine weiteren Maßnahmen erforderlich; es genügt also in diesem Falle eine "stand alone"-Technik, bei der ausschließlich die Zwischenwirbelimplantate 1, 1' eingesetzt werden. Andernfalls erfolgt zusätzlich eine Vergurtung mittels eines Fixateur Interne.

## Patentansprüche

1. Zwischenwirbelimplantat mit
a) einem Kopf (2), der einen konischen Abschnitt (13) und ein Außengewinde (14) trägt;
b) einem an den Kopf (2) angeformten Zentralabschnitt (5, 6, 7, 8), der ebenfalls ein Außengewinde (16) trägt;
c) einer an dem Zentralabschnitt (5, 6, 7, 8) angeformten Grundstruktur (3),
wobei
d) der Zentralabschnitt (5, 6, 7, 8) von einer Mehrzahl sich vom Kopf (2) zur Grundstruktur (3) erstreckender Streben gebildet ist, so daß zwischen dem Kopf (2), den Streben (5, 6, 7, 8) und der Grundstruktur (3) großflächige Fenster (9, 10, 11, 12) offen bleiben,
e) das Außengewinde (14) des Kopfes (2) auf dem koni- sehen Abschnitt (13) ausgebildet ist;
**dadurch gekennzeichnet, daß**
f) der Kopf (2) einen äußeren Endbereich aufweist, wobei der äußere Endbereich des Kopfes (2) von einer Schutzerhebung (15) gebildet ist, die von außen gesehen konvex gekrümmt und frei von scharfen Kanten ist;
g) sich der konische Abschnitt (13) zum Endbereich des Kopfes (2) hin verjüngt, und daß
h) die Mantelflache des Zentralabschnittes mit der Mantelflache des konischen Abschnittes des Kopfes einen Winkel einschließt, der größer als 0° ist.

2. Zwischenwirbelimplantat nach Anspruch 1, **dadurch**
**gekennzeichnet, daß** der konische Abschnitt (13) des Kopfes (2) eine axiale Erstreckung (D₄) von mindestens 7 mm besitzt.

3. Zwischenwirbelimplantat nach Anspruch 1 oder 2,
**dadurch gekennzeichnet, daß** das Außengewinde (14) auf dem Kopf (2) mindestens drei volle Gewindegänge aufweist.

4. Zwischenwirbelimplantat nach einem der vorhergehenden Ansprüche, **dadurch gekennzeichnt, daß** die Schutzerhebung (15) des Kopfes (2) eine axiale Erstreckung (D₃) von mindestens 5 mm aufweist.

5. Zwischenwirbelimplantat nach einem der vorhergehenden Ansprüche, **dadurch gekennzeichnet, daß** die Schutzerhebung (15) die Form einer geschlossenen Kappe besitzt.

6. Zwischenwirbelimplantat nach einem der Ansprüche
1 bis 4, **dadurch gekennzeichnet, daß** die Schutzerhebung ringförmig ist und eine mittlere Durchgangsöffnung aufweist.

7. Zwischenwirbelimplantat nach einem der vorhergehenden Ansprüche, **dadurch gekennzeichnet, daß** die Grundstruktur (3) eine axiale Erstreckung (D₅) von mindestens 5 mm aufweist.

8. Zwischenwirbelimplantat nach einem der vorhergehenden Ansprüche, **dadurch gekennzeichnet, daß** die Grundstruktur (3) eine Platte ist.

9. Zwischenwirbelimplantat nach einem der vorhergehenden Ansprüche, **dadurch gekennzeichnet, daß** an der Grundstruktur (3) eine Angriffseinrichtung (18, 19) für ein Eindrehinstrument vorgesehen ist.

10. Zwischenwirbelimplantat nach einem der vorhergehenden Ansprüche, **dadurch gekennzeichnet, daß** der von der konischen Mantelfläche des konischen Abschnittes (13) des Kopfes (2) und von einer gedachten koaxialen Kreiszylinder-Mantelfläche eingeschlossene Winkel (α) zwischen 10° und 20°, bevorzugt bei 15°, liegt.

11. Zwischenwirbelimplantat nach einem der vorhergehenden Ansprüche, **dadurch gekennzeichnet, daß** der Zentralabschnitt (5, 6, 7, 8) und die Grundstruktur (3) gegensinnig zum konischen Abschnitt (13) des Kopfes (2) konisch sind.

12. Zwischenwirbelimplantat nach Anspruch 11, **dadurch**
**gekennzeichnet, daß** der von der konischen Mantelfläche des Zentralabschnittes (5, 6, 7, 8) und der Grundstruktur (3) und von einer gedachten koaxialen Kreiszylinder-Mantelfläche eingeschlossene Winkel (β) etwa 3° beträgt.

13. Zwischenwirbelimplantat nach Anspruch 11, **dadurch**
**gekennzeichnet, daß** der von der konischen Mantelfläche des Zentralabschnittes (5, 6, 7, 8) und der Grundstruktur (3) und von einer gedachten koaxialen Kreiszylinder-Mantelfläche eingeschlossene Winkel (β) etwa 6° beträgt.

14. Zwischenwirbelimplantat nach einem der vorhergehenden Ansprüche, **dadurch gekennzeichnet, daß** es in mindestens einem axialen Bereich eine nicht rotationssymmetrische Querschnittsform aufweist.

15. Zwischenwirbelimplantat nach Anspruch 14, **dadurch**
**gekennzeichnet, daß** die Grundstruktur (3) eine nicht rotationssymmetrische Querschnittsform aufweist.

16. Zwischenwirbelimplantat nach einem der vorhergehenden
Ansprüche, **dadurch gekennzeichnet, daß** die Schutzerhebung (15) poliert ist.

## Claims

1. Intervertebral implant comprising
a) a head (2), which carries a conical portion (13) and an external thread (14);
b) a central portion (5, 6, 7, 8) formed on the head (2) and also carrying an external thread (16);
c) a basic structure (3) formed on the central portion (5, 5, 7, 8),
wherein
d) the central portion (5, 6, 7, 8) is formed by a plurality of struts extending from the head (2) to the basic structure (3), so that large-area windows (9, 10, 11, 12) remain open between the head (2), the struts (5, 6, 7, 8) and the basic structure (3),
e) the external thread (14) of the head (2) is formed on the conical portion (13);
**characterised in that**
f) the head (2) comprises an outer end region, wherein the outer end region of the head (2) is formed by a protective elevation (15) which is convexly curved, viewed from the outside, and is free from sharp edges;
g) the conical portion (13) tapers down towards the outer end region of the head (2), and **in that**
h) the circumferential surface of the central portion forms with the circumferential surface of the conical portion of the head an angle which is greater than 0°.

2. Intervertebral implant according to claim 1, **characterised in that** the conical portion (13) of the head (2) has an axial extension (D₄) of at least 7 mm.

3. Intervertebral implant according to claim 1 or 2,
**characterised in that** the external thread (14) on the head (2) comprises at least three full turns.

4. Intervertebral implant according to any one of the
preceding claims, **characterised in that** the protective elevation (15) of the head (2) comprises an axial extension (D₃) of at least 5 mm.

5. Intervertebral implant according to any one of the preceding claims, **characterised in that** the protective elevation (15) has the form of a closed cap.

6. Intervertebral implant according to any of claims 1
to 4, **characterised in that** the protective elevation is annular and has a central through-opening.

7. Intervertebral implant according to any one of the
preceding claims, **characterised in that** the basic structure (3) has an axial extension (D₅) of at least 5 mm.

8. Intervertebral implant according to any one of the
preceding claims, **characterised in that** the basic structure (3) is a plate.

9. Intervertebral implant according to any one of the
preceding claims, **characterised in that** an application device (18, 19) for a screw-in instrument is provided on the basic structure (3).

10. Intervertebral implant according to any one of the preceding claims, **characterised in that** the angle (α) enclosed by the conical circumferential surface of the conical portion (13) of the head (2) and by an imaginary coaxial circumferential surface of a circular cylinder is between 10° and 20°, and is preferably 15°.

11. Intervertebral implant according to any one of the
preceding claims, **characterised in that** the central portion (5, 6, 7, 8) and the basic structure (3) are conical in the opposite direction to the conical portion (13) of the head (2).

12. Intervertebral implant according to claim 11, **characterised in that** the angle (β) enclosed by the conical circumferential surface of the central portion (5, 6, 7, 8) and the basic structure (3) and by an imaginary coaxial circumferential surface of a circular cylinder is about 3°.

13. Intervertebral implant according to claim 11, **characterised in that** the angle (β) enclosed by the conical circumferential surface of the central portion (5, 6, 7, 8) and the basic structure (3) and by an imaginary coaxial circumferential surface of a circular cylinder is about 6°.

14. Intervertebral implant according to any one of the
preceding claims, **characterised in that** in at least one axial region it has a non-rotationally symmetrical cross-sectional form.

15. Intervertebral implant according to claim 14, **characterised in that** the basic structure (3) has a non-rotationally symmetrical cross-sectional form.

16. Intervertebral implant according to any one of the
preceding claims, **characterised in that** the protective elevation (15) is polished.

## Revendications

1. Implant intervertébral comprenant
a) une tête (2) portant une région conique (13) et un filetage extérieur (14) ;
b) une région centrale (5, 6, 7, 8) faisant corps avec ladite tête (2) et pareillement munie d'un filetage extérieur (16) ;
c) une structure d'embase (3) faisant corps avec ladite région centrale (5, 6, 7, 8),
sachant que
d) ladite région centrale (5, 6, 7, 8) est constituée d'une pluralité de membrures s'étendant depuis la tête (2) jusqu'à la structure d'embase (3), de sorte que des fenêtres (9, 10, 11, 12) à grande superficie demeurent ouvertes entre ladite tête (2), lesdites membrures (5, 6, 7, 8) et ladite structure d'embase (3),
e) le filetage extérieur (14) de la tête (2) est façonné sur la région conique (13),
**caractérisé par** le fait
f) que ladite tête (2) comporte une région extrême extérieure, ladite région extrême extérieure de la tête (2) étant formée d'une excroissance protectrice (15) qui, observée de l'extérieur, offre une courbure convexe et est exempte d'arêtes vives ;
g) que la région conique (13) s'amenuise en direction de ladite région extrême de ladite tête (2) ; et
h) que la surface périphérique de la région centrale décrit un angle supérieur à 0° avec la surface périphérique de la région conique de la tête.

2. Implant intervertébral selon la revendication 1, **caractérisé par le fait que** la région conique (13) de la tête (2) présente une étendue axiale (D₄) d'au moins 7 mm.

3. Implant intervertébral selon la revendication 1 ou 2, **caractérisé par le fait que** le filetage extérieur (14), situé sur la tête (2), compte au moins trois pas de filetage intégraux.

4. Implant intervertébral selon l'une des revendications précédentes, **caractérisé par le fait que** l'excroissance protectrice (15) de la tête (2) présente une étendue axiale (D₃) d'au moins 5 mm.

5. Implant intervertébral selon l'une des revendications précédentes, **caractérisé par le fait que** l'excroissance protectrice (15) revêt la forme d'un capuchon fermé.

6. Implant intervertébral selon l'une des revendications 1 à 4, **caractérisé par le fait que** l'excroissance protectrice est de forme annulaire et comporte un orifice central de passage.

7. Implant intervertébral selon l'une des revendications précédentes, **caractérisé par le fait que** la structure d'embase (3) présente une étendue axiale (D₅) d'au moins 5 mm.

8. Implant intervertébral selon l'une des revendications précédentes, **caractérisé par le fait que** la structure d'embase (3) est une platine.

9. Implant intervertébral selon l'une des revendications précédentes, **caractérisé par le fait qu'**un dispositif (18, 19) de venue en prise, destiné à un instrument d'insertion par rotation, est prévu sur la structure d'embase (3).

10. Implant intervertébral selon l'une des revendications précédentes, **caractérisé par le fait que** l'angle (α), décrit par la surface d'enveloppe conique de la région conique (13) de la tête (2) et par une surface d'enveloppe cylindrique droite, imaginaire et coaxiale, est compris entre 10° et 20°, et avoisine 15° de préférence.

11. Implant intervertébral selon l'une des revendications précédentes, **caractérisé par le fait que** la région centrale (5, 6, 7, 8) et la structure d'embase (3) présentent une conicité en sens inverse de celle de la région conique (13) de la tête (2).

12. Implant intervertébral selon la revendication 11, **caractérisé par le fait que** l'angle (β), décrit par la surface d'enveloppe conique de la région centrale (5, 6, 7, 8) et de la structure d'embase (3), et par une surface d'enveloppe cylindrique droite, imaginaire et coaxiale, mesure environ 3°.

13. Implant intervertébral selon la revendication 11, **caractérisé par le fait que** l'angle (β), décrit par la surface d'enveloppe conique de la région centrale (5, 6, 7, 8) et de la structure d'embase (3), et par une surface d'enveloppe cylindrique droite, imaginaire et coaxiale, mesure environ 6°.

14. Implant intervertébral selon l'une des revendications précédentes, **caractérisé par le fait qu'**il possède, au moins dans une région axiale, une configuration de section transversale dépourvue de symétrie de révolution.

15. Implant intervertébral selon la revendication 14, **caractérisé par le fait que** la structure d'embase (3) possède une configuration de section transversale dépourvue de symétrie de révolution.

16. Implant intervertébral selon l'une des revendications précédentes, **caractérisé par le fait que** l'excroissance protectrice (15) est polie.
